(19) Europäisches Patentamt
European Patent Office
Office européen des brevets

(11) Veröffentlichungsnummer: **0 296 545 B1**

# (12) EUROPÄISCHE PATENTSCHRIFT

(45) Veröffentlichungstag der Patentschrift: **04.03.92**

(51) Int. Cl.[5]: **C07C 229/30**, C12P 1/02, C12P 13/04, A61K 31/20, //(C12P1/02,C12R1:68)

(21) Anmeldenummer: **88109887.5**

(22) Anmeldetag: **22.06.88**

## (54) Ein neues Antibiotikum, Fumifungin, ein mikrobielles Verfahren zu seiner Herstellung und seine Verwendung als Arzneimittel.

(30) Priorität: **25.06.87 DE 3720981**

(43) Veröffentlichungstag der Anmeldung:
**28.12.88 Patentblatt 88/52**

(45) Bekanntmachung des Hinweises auf die Patenterteilung:
**04.03.92 Patentblatt 92/10**

(84) Benannte Vertragsstaaten:
**AT BE CH DE ES FR GB GR IT LI LU NL SE**

(56) Entgegenhaltungen:

**CHEMICAL ABSTRACTS, Band 107, Nr. 13, 28. September 1987, Columbus, Ohio, USA; MUKHOPADHYAY Triptikumar et al: "Fumifungin, a new antifungal antibiotic from Aspergillus fumigatus Fresenius 1863", S. 333, Spalte 1, Zusammenfassung Nr. 112297m**

(73) Patentinhaber: **HOECHST AKTIENGESELLSCHAFT
Postfach 80 03 20
W-6230 Frankfurt am Main 80(DE)**

(72) Erfinder: **Mukhopadhyay, Triptikumar, Dr.,
M-24, Hoechst Ouarters, Darga Road, Mulund(West),
Bombay 400 082(IN)**
Erfinder: **Roy, Kirity, Dr.
G-31, Hoechst Ouarters, Darga Road, Mulund (West),
Bombay 400 082(IN)**
Erfinder: **Fehlhaber, Hans-Wolfgang, Dr.,
Thomas-Mann-Strasse 5a,
W-6270 Idstein/Taunus(DE)**
Erfinder: **Rupp, Richard Helmut, Dr.,
Roederweg 16a,
W-6240 Koenigstein/Taunus(DE)**
Erfinder: **Ganguli, Bimal Naresh, Dr.,
"Saurayuth" 173 Central Avenue, Chembur,
Bombay 400 071(IN)**

EP 0 296 545 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft ein neues, als Fumifungin bezeichnetes Antibiotikum sowie ein Verfahren zu dessen Herstellung aus der Pilz-Kultur Nr. Y-83,0405 und dessen Mutanten und Varianten.

Fumifungin kann bezeichnet werden als 2-Amino-4-acetoxy-3,5,14 -trihydroxy-$\Delta^6$-eikosensäure und besitzt die folgende Strukturformel:

$$H_3C(CH_2)_5 - \underset{OH}{CH}(CH_2)_6 - CH = CH - \underset{OH}{CH} - \underset{OAc}{CH} - \underset{OH}{CH} - \underset{NH_2}{CH} - COOH .$$

Weitere, aber von Fumifungin verschiedene Antibiotika aus dieser Gruppe, über die in der Literatur berichtet wird, sind Thermozymocidin und/oder Myriocin.

Thermozymocidin wird in Tetrahedron 28, 5493, 1972, Chemical Communications 788, 1973, Tetrahedron Letters 211, 1978 und im belgischen Patent Nr. 796 682 beschrieben. Myriocin wurde im Journal of Antibiotics, 25, 109, 1972, Journal of Organic Chemistry, 38, 1253, 1973 und in U.S. Patent Nr. 3 928 572 beschrieben.

Beschreibungen des Thermozymocidins und Myriocins finden sich ferner im CRC-Handbook of Antibiotic Compounds, Vol. VI, S. 391-418, im Abschnitt "Fatty Acid Derivatives" sowie in "Fungal Metabolites 11" von W. B. Turner & D. C. Aldridge, Academic Press 1983, S. 173-175. Der Thermozymocidin produzierende Organismus wurde als thermophiler Ascomycet Myriococcum albomyces beschrieben.

Nach allen vorliegenden Kenndaten unterscheidet sich Fumifungin jedoch deutlich von Thermozymocidin und Myriocin und allen übrigen bekannten Antibiotika.

Die zur Produktion von Fumifungin verwendete Pilzkultur Nr. Y-83,0405 wurde aus einer im Himalaya in Indien gezogenen Bodenprobe isoliert und nach bekannten Methoden durch ihre physiologischen, biochemischen und morphologischen Eigenschaften als Aspergillus fumigatus Fresenius 1863 identifiziert.

Sie wurde am 23.6.1987 bei der "Deutschen Sammlung von Mikroorganismen" (Göttingen) unter der Eingangsnummer DSM 4152 hinterlegt.

Gegenstand vorliegender Erfindung ist auch ein Verfahren zur Herstellung des neuen Antibiotikums Fumifungin. Dieses Verfahren besteht in der Kultivierung der Pilzkultur Nr. Y-83,0405 oder deren Mutanten und Varianten unter aeroben Bedingungen auf einem Nährboden, der Kohlenstoff- und Stickstoffquellen, anorganische Nährsalze und Spurenelemente enthält und der Isolierung und Reinigung dieses Antibiotikums aus der Kulturbrühe.

Als Kohlenstoffquellen kommen Glucose, Rohrzucker, Stärke oder Dextrin in Frage. Glucose wird als Kohlenstoffquelle bevorzugt. Als Stickstoffquellen kommen Sojabohnenmehl, Trypton, Hefeextrakt, Rindfleischextrakt, Malzextrakt, Maisquellwasser, Pepton oder anorganische Stoffe wie Ammoniumsalze in Betracht. Malzextrakt oder eine Kombination von Malzextrakt mit anderen Stickstoffquellen wird als solche bevorzugt. Bei den anorganischen Nährsalzen kann es sich um Natriumchlorid, Kaliumhydrogen/Dihydrogenphosphat oder Calciumcarbonat handeln. Als Spurenelemente können Salze des Eisens, Mangans, Kupfers, Zinks, Kobalts oder anderer solcher Schwermetalle vorliegen.

Die Züchtung der Kultur Nr. Y-83,0405 kann bei Temperaturen zwischen 24 °C und 30 °C und einem pH zwischen 6,0 und 8,0 erfolgen. Vorzugsweise wird die Kultur Nr. Y-83,0405 bei 26 °C (± 1 °C) und pH 6,5 gezüchtet.

Die Kultivierung wird 66 bis 96 Stunden lang durchgeführt, nach welcher Zeit man eine optimale Ausbeute des erfindungsgemäßen Antibiotikums erhält. Die Fermentation erfolgt vorzugsweise 90 Stunden lang unter Submersbedingungen in Schüttelkolben. Der Fortgang der Fermentation und der Bildung des erfindungsgemäßen Fumifungins läßt sich durch Messung der Bioaktivität der Kulturbrühe gegen Bacillus subtilis, Candida albicans und Aspergillus niger nach der bekannten Agarplatten-Diffusionsbestimmungsmethode verfolgen.

In der entstandenen Kulturbrühe liegt Fumifungin sowohl im Kulturfiltrat als auch im Mycelkuchen vor. Fumifungin wird vorzugsweise aus dem Kulturfiltrat isoliert, die von dem Mycel durch Zentrifugieren abgetrennt wird. Es läßt sich nach einer oder mehreren bekannten Methoden, wie Extraktion unter Verwendung von mit Wasser nicht mischbaren Lösungsmitteln wie n-BuOH oder Adsorption an Adsorptionsmitteln wie Aktivkohle, Diaion HP-20® oder Amberlit XAD-2®, aus dem Kulturfiltrat gewinnen. Die bevorzugte Methode ist Adsorption an Diaion HP-20® und nachfolgende Desorption der Verbindung mit geeigneten organischen Lösungsmitteln. Die dafür in Frage kommenden organischen Lösungsmittel sind

Methanol, Aceton oder Acetonitril sowie wäßrige Kombinationen dieser Lösungsmittel. Für das erfindungsgemäße Verfahren wird stufenweises Eluieren mit (1) MeOH:$H_2O$ (1:1) und danach MeOH:$H_2O$ (8:2) als Lösungsmitteln bevorzugt. Die weitere Verarbeitung des durch Eluieren mit MeOH:$H_2O$ (8:2) aus Diaion HP-20® erhaltenen Fumifungins kann etwa durch Eindampfen des Gesamtvolumens zur Trockne oder Extraktion der Eluate mit n-BuOH oder Readsorption der verdünnten Eluate an Harzen wie Diaion HP-20® oder Amberlit XAD-2® erfolgen. Die bevorzugte Methode besteht darin, daß man die Eluate im Vakuum eingengt, um einen Großteil des organischen Lösungsmittels zu entfernen, das so erhaltene Konzentrat mit entmineralisiertem Wasser verdünnt und die entstandene Lösung an Diaion HP-20® readsorbiert und danach mit MeOH:$H_2O$ (6:4) und dann 100%igem MeOH desorbiert. Entfernung des Lösungsmittels aus den aktiven Eluaten liefert rohes Fumifungin. Die Endreinigung läßt sich durch Chromatographie an Substraten wie Kieselgel, modifizierten Kieselgelen, Zellulose oder LH-20 oder durch Kristallisieren des rohen Fumifungins aus organischen Lösungsmitteln oder deren wäßrigen Gemischen bzw. eine Kombination dieser Methoden erreichen. Bei der bevorzugten Methode chromatographiert man das rohe Fumifungin über eine mit modifiziertem Kieselgel (Dimethyloctadecyl-silyliertes Kieselgel 50 $\mu$), das im folgenden als RP-18 bezeichnet wird, gefüllte Säule und eluiert unter Druck nach der als Mitteldruckflüssigkeitschromatographie (medium pressure liquid chromatography - MPLC) bekanntem Methode.

Unter den verschiedenen Lösungsmitteln zum Eluieren aus RP-18, wie Methanol, Acetonitril oder Aceton bzw. deren wäßrigen Gemischen, verwendet man zum stufenweisen Eluieren vorzugsweise zunächst MeOH:$H_2O$ (6:4) und danach MeOH:$H_2O$ (7:3). Die Entfernung der Lösungsmittel aus den aktiven Eluaten im Vakuum und vollständige Lyophilisierung liefert Fumifungin als farblosen Feststoff.

Die nachfolgenden Beispiele dienen der Erläuterung der vorliegenden Erfindung:

**Beispiel I: Isolierung der Kultur Y-83,0405 aus Boden**

Zusammensetzung des Nährmediums zur Isolierung

| | |
|---|---|
| Glucose | 40 g |
| Pepton | 10 g |
| Agar | 15 g |
| Entmineralisiertes Wasser | 1 Liter |
| Chloramphenicol | 0,05 g |
| Cycloheximid | 0,2 g |
| pH | 9,0 |

Bei der Herstellung des obigen Mediums werden Chloramphenicol und Cycloheximid zuletzt zugesetzt. Das in 10 ml 95%igem Äthanol gelöste Chloramphenicol wird dem Medium zugesetzt und gut eingemischt. Danach gibt man eine Cycloheximidlösung in 10 ml Aceton dazu und vermischt gründlich mit dem Medium. Dieses wird 10 Minuten lang bei einer Temperatur von 121 ° C autoklaviert. Der pH betrugt 9,0 vor dem Autoklavieren und 7,0 danach.

Je 50 ml des sterilisierten, auf 45 ° C abgekühlten Mediums gießt man in 150 mm-Petrischalen und läßt erstarren.

Die Pilzkultur Nr. Y-83,0405 wurde aus der Bodenprobe aus dem Himalaya nach der Bodenverdünnungsmethode und Ausstreichen auf dem obigen Medium in bekannter Weise isoliert.

**Beispiel II: Stammhaltung der Kultur Y-83,0405**

Die Kultur Nr. Y-83,0405 wird auf Glucoseagarmedium nach Sabouraud der folgenden Zusammensetzung gehalten:

| | |
|---|---|
| Glucose | 40 g |
| Pepton | 10 g |
| $Na_2HPO_4$ | 1 g |
| Agar | 15 g |
| Entmineralisiertes Wasser | 1 Liter |
| pH | 6,5 |

Nach vollständiger Auflösung der Bestandteile durch Erwärmen wird das Medium über Reagenzgläser verteilt und dann 20 Minuten lang bei 121 ° C sterilisiert. Man läßt die Reagenzgläser abkühlen und das Medium erstarren. Schrägagar wird mit Sporen der Kultur Nr. Y-83,0405 beimpft und bei 26 ° C (± 1 ° C) inkubiert, bis gute Sporenbildung zu beobachten ist. Die Kulturen mit guter Sporenbildung werden im Kühlschrank aufbewahrt.

**Beispiel III: Fermentation der Kultur Y-83,0405 in Schüttelkolben**

Zusammensetzung des Impfkulturmediums

| | |
|---|---|
| Lösliche Stärke | 15 g |
| Sojabohnenmehl | 15 g |
| Glucose | 5 g |
| $CaCO_3$ | 2 g |
| NaCl | 5 g |
| Hefeextrakt | 2 g |
| Maisquellwasser | 1 g |
| Entmineralisiertes Wasser | 1 Liter |
| pH | 6,5 |

Je 100 ml des obigen Impfkulturmediums werden über weithalsige 500 ml-Erlenmeyerkolben verteilt und bei 121 ° C 20 Minuten lang sterilisiert. Die Kolben werden abgekühlt und dann mit einigen Platinösen voll der oben erwähnten Kultur mit guter Sporenbildung beimpft und 60 Stunden lang bei 240 U.p.M. bei 26 ° C (± 1 ° C) geschüttelt, wobei gutes Wachstum beobachtet wird. Die so erhaltene Impfkultur wird zum Beimpfen des Produktionsmediums der folgenden Zusammensetzung verwendet.

Zusammensetzung des Produktionsmediums:

| | |
|---|---|
| Glucose | 10 g |
| Malzextrakt | 20 g |
| Pepton | 10 g |
| $Na_2HPO_4$ | 1 g |
| $ZnSO_4 \cdot 7H_2O$ | 0,22 mg |
| $CaCl_2$ | 0,55 mg |
| $MnCl_2 \cdot 4H_2O$ | 0,5 mg |
| $FeSO_4 \cdot 5H_2O$ | 0,16 mg |
| $CoCl_2 \cdot 6H_2O$ | 0,16 mg |
| Entmineralisiertes Wasser | 1 Liter |
| pH vor Sterilisation | 6,5 |
| pH nach Sterilisation | 6,3 |

Je 200 ml des obigen Produktionsmediums werden über 1 Liter-Erlenmeyerkolben verteilt und 20 Minuten lang bei 121 ° C sterilisiert. Die Kolben wurden abgekühlt und dann mit dem obigen Impfkulturmedium (1% V/V) beimpft. Die Fermentation erfolgt 90 Stunden lang auf einer Rotationsschüttelmaschine bei 220 U.p.M. und einer Temperatur von 26 ° C (± 1 ° C).

Die Fumifunginproduktion wird durch das Aktivitätsprofil gegen Bacillus subtilis, Candida albicans und Aspergillus niger geprüft. Nach dem Ernten wird die Kulturbrühe zentrifugiert und das Fumifungin aus dem Kulturfiltrat isoliert und wie in Beispiel IV beschrieben gereinigt.

**Beispiel IV: Isolierung und Reinigung des Fumifungins**

Ungefähr 60 Liter der Fermentationsbrühe werden zur Abtrennung des Mycels vom Kulturfiltrat zentrifugiert.

Das Kulturfiltrat (55 Liter) wird auf pH 5,9 gestellt und durch eine mit Diaion HP-20® (1,8 Liter) gefüllte Säule geleitet. Die Säule wird mit entmineralisiertem Wasser gewaschen (7 Liter). Durch Eluieren mit 50%igem Methanol in Wasser (20 Liter) werden stark gefärbte, inaktive Verunreinigungen entfernt.

Die Diaion HP-20®-Säule wird dann mit 80%igem Methanol in Wasser eluiert. Die ersten Fraktionen (2 x 800 ml) sind inaktiv, aber die nachfolgenden Fraktionen (12 x 800 ml) zeigen Aktivität. Die aktiven Eluate werden bei vermindertem Druck auf 7 Liter Konzentrat eingeengt. Fortgesetztes Eluieren mit 80%igem Methanol in Wasser (10 x 800 ml) ergibt keine weitere Aktivität. Das Konzentrat wird durch Zusatz von 15 Liter entmineralisiertem Wasser verdünnt. Die verdünnte Lösung wird nochmals durch Diaion HP-20® (1 Liter) geleitet und mit 60%igem Methanol in Wasser (10 Liter) gewaschen. Das Methanol/Wasser wird völlig aus der Säule abgezogen, und diese wird dann mit Methanol (4 Liter) eluiert. Entfernung des Lösungsmittels bei vermindertem Druck und nachfolgende Lyophilisierung ergeben 1,5 g rohes Fumifungin.

Auf das rohe Fumifungin (600 mg) wird Mitteldruckflüssigkeitschromatographie unter Verwendung von RP-18 als Umkehrphasenträgermaterial angewendet. Die Säule wird mit 60%igem Methanol in Wasser bei

einer Durchflußmenge von 11 ml/Min. eluiert, bis ein Liter Eluat aufgelaufen ist. Das Eluiermittel wird dann zu 70% Methanol in Wasser gewechselt. Die aktiven Fraktionen werden vereinigt, bei vermindertem Druck eingeengt und lyophilisiert, wobei man 100 mg reines, farbloses Fumifungin erhält.

Die physikalisch-chemischen Eigenschaften des Fumifungins sind in Tabelle I angeführt.

Tabelle I

| Physikalisch-chemische Eigenschaften des Fumifungins | | | | | | |
|---|---|---|---|---|---|---|
| 1. Aussehen | Farbloses Pulver | | | | | |
| 2. Löslichkeit | Methanol, DMSO | | | | | |
| 3. Elementaranalyse | Ber. | C,<br>H,<br>N, | 61,25%,<br>9,51%<br>3,20% | gef. | C,<br>H,<br>N, | 58,9%<br>9,5%<br>3,2% |
| 4. Summenformel | $C_{22}H_{41}NO_7$ | | | | | |
| 5. Molekulargewicht | 431 | | | | | |
| 6. Schmelzpunkt | 108 ° C | | | | | |
| 7. Farbreaktion | a) $KMnO_4$ -Entfärbung | | | | | |
| | b) Ninhydrin - Violettfärbung | | | | | |
| 8. UV (MeOH) | Endabsorption | | | | | |
| 9. IR (Nujol) | 3300, 3000, 1725, 1640, 1570, 1400, 1350, 1240, 1090, 975, 770, 730 $cm^{-1}$. | | | | | |
| 10. $^1$H-NMR ($CD_3OD$) | 0,9, t (J = 6 Hz), 3H | | | | | |
| | 1,2-1,5, m breit, 20H | | | | | |
| | 2,05, s , 3H | | | | | |
| | 2,1, m, 2H | | | | | |
| | 3,5, s breit, 1H | | | | | |
| | 3,76, d(J = 4 Hz), 1H | | | | | |
| | 3,82, d(J = 6 Hz), 1H | | | | | |
| | 4,16, dd (J = 2 & 4 Hz), 1H | | | | | |
| | 5,33-5,4, m, 2H | | | | | |
| | 5,83,-5,95, m, 1H | | | | | |

Die neue Verbindung Fumifungin besitzt bakterien- und pilztötende Eigenschaften. Die zur Hemmung des Wachstums verschiedener Bakterien- und Pilzstämme erforderlichen minimalen Hemmkonzentrationen (MHK) von Fumifungin sind in der folgenden Tabelle II aufgeführt:

Tabelle II

| MHK-Werte für Fumifungin | | |
|---|---|---|
| Lfd. Nummer | Teststämme | MHK-Werte in g/l |
| 1. | Candida albicans | 62,5 |
| 2. | Saccharomyces cerevisiae | 62,5 |
| 3. | Wild Yeast | 62,5 |
| 4. | Aspergillus niger | >7,8 |
| 5. | Penicillium digitatum | >7,8 |
| 6. | Trichophyton mentagrophytes | >15,6 |
| 7. | Botrytis cinerea | 250 |
| 8. | Fusarium culmorum | 250 |
| 9. | Alternaria solani | 31,2 |
| 10. | Cercospora beticola | 0,9 |
| 11. | Cladosporium resinae | >0,9 |
| 12. | Piricularia oryzae | 125 |

**Patentansprüche**

**Patentansprüche für folgende Vertragsstaaten : AT, BE, CH, DE, FR, GB, IT, LI, LU, NL, SE**

**1.** Eine Verbindung der Formel

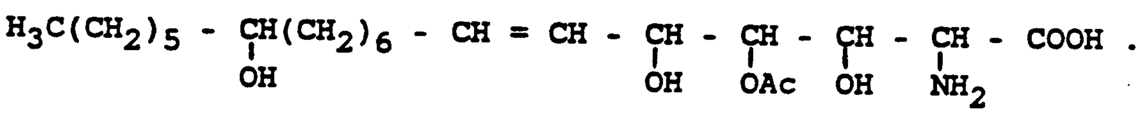

**2.** Verfahren zur Herstellung der Verbindung gemäß Anspruch 1, dadurch gekennzeichnet, daß man die Pilzkultur Aspergillus fumigatus Nr. Y-83,0405 (DSM 4152) unter aeroben Bedingungen auf einem Nährboden, der Kohlenstoffquellen, Stickstoffquellen, anorganische Nährsalze und Spurenelemente enthält, bei einer Temperatur zwischen 24 und 30 ° C und einem pH zwischen 6,0 und 8,0 66 bis 96 Stunden lang kultiviert und die Verbindung in üblicher Weise aus der Kulturbrühe isoliert und reinigt.

**3.** Verfahren nach Anspruch 2, dadurch gekennzeichnet, daß bei 26 ° C (± 1 ° C) und pH 6,5 kultiviert wird.

**4.** Verfahren nach Anspruch 2 oder 3, dadurch gekennzeichnet, daß die Kultivierung 90 Stunden lang durchgeführt wird.

**5.** Verfahren nach einem der Ansprüche 2 bis 4, dadurch gekennzeichnet, daß die Fermentation als submerse Fermentation durchgeführt wird.

**6.** Arzneimittel, gekennzeichnet durch einen Gehalt an einer Verbindung gemäß Anspruch 1 neben üblichen Hilfs- und/oder Trägerstoffen.

**7.** Verwendung einer Verbindung gemäß Anspruch 1 zur Herstellung von Arzneimitteln mit antibiotischer Wirkung.

**8.** Aspergillus fumigatus Y-83,0405 (DSM 4152).

**Patentansprüche für folgende Vertragsstaaten : ES, GR**

**1.** Verfahren zur Herstellung einer Verbindung der Formel I

$$H_3C(CH_2)_5-\underset{OH}{CH}(CH_2)_6-CH=CH-\underset{OH}{CH}-\underset{OAc}{CH}-\underset{OH}{CH}-\underset{NH_2}{CH}-COOH \qquad I$$

dadurch gekennzeichnet, daß man die Pilzkultur Aspergillus fumigatus Nr. Y-83,0405 (DSM 4152) unter aeroben Bedingungen auf einem Nährboden, der Kohlenstoffquellen, Stickstoffquellen, anorganische Nährsalze und Spurenelemente enthält, bei einer Temperatur zwischen 24 und 30 ° C und einem pH zwischen 6,0 und 8,0 66 bis 96 Stunden lang kultiviert und die Verbindung in üblicher Weise aus der Kulturbrühe isoliert und reinigt.

**2.** Verfahren nach Anspruch 1, dadurch gekennzeichnet, daß bei 26 ° C (± 1 ° C) und pH 6,5 kultiviert wird.

**3.** Verfahren nach Anspruch 1 oder 2, dadurch gekennzeichnet, daß die Kultivierung 90 Stunden lang durchgeführt wird.

**4.** Verfahren nach einem der Ansprüche 1 bis 3, dadurch gekennzeichnet, daß die Fermentation als submerse Fermentation durchgeführt wird.

**5.** Verfahren zur Herstellung von Arzneimitteln, dadurch gekennzeichnet, daß eine Verbindung der Formel I gemäß Anspruch 1 mit üblichen Hilfs- und/oder Trägerstoffen in eine geeignete Darreichungsform gebracht wird.

**6.** Verwendung einer Verbindung gemäß Anspruch 1 zur Herstellung von Arzneimitteln mit antibiotischer Wirkung.

**Claims**

**Claims for the following Contracting States : AT, BE, CH, DE, FR, GB, IT, LI, LU, NL, SE**

**1.** A compound of the formula

$$H_3C(CH_2)_5-\underset{OH}{CH}(CH_2)_6-CH=CH-\underset{OH}{CH}-\underset{OAc}{CH}-\underset{OH}{CH}-\underset{NH_2}{CH}-COOH$$

**2.** A process for the preparation of the compound as claimed in claim 1, which comprises cultivation of the fungus culture Aspergillus fumigatus No. Y-83,0405 (DSM 4152) under aerobic conditions on a nutrient medium which contains carbon sources, nitrogen sources, inorganic nutrient salts and trace elements, at a temperature between 24 and 30 ° C and at a pH between 6.0 and 8.0 for 66 to 96 hours, and isolation and purification of the compound from the culture broth in a customary manner.

**3.** The process as claimed in claim 2, wherein cultivation is at 26 ° C (± 1 ° C) and pH 6.5.

**4.** The process as claimed in claim 2 or 3, wherein the cultivation is carried out for 90 hours.

**5.** The process as claimed in one of claims 2 to 4, wherein the fermentation is carried out as a submerged fermentation.

**6.** A pharmaceutical which contains a compound as claimed in claim 1, together with customary auxiliaries and/or vehicles.

**7.** The use of a compound as claimed in claim 1 for the preparation of pharmaceuticals having an antibiotic action.

**8.** Aspergillus fumigatus Y-83,0405 (DSM 4152).

**Claims for the following Contracting States : ES, GR**

**1.** A process for the preparation of a compound of the formula I

```
H3C(CH2)5-CH(CH2)6-CH=CH-CH-CH-CH-CH-COOH
          |                 |  |   |  |
          OH                OH OAcOH NH2
```

which comprises cultivation of the fungus culture Aspergillus fumigatus No. Y-83,0405 (DSM 4152) under aerobic conditions on a nutrient medium which contains carbon sources, nitrogen sources, inorganic nutrient salts and trace elements, at a temperature between 24 and 30 ° C and at a pH between 6.0 and 8.0 for 66 to 96 hours, and isolation and purification of the compound from the culture broth in a customary manner.

**2.** The process as claimed in claim 1, wherein cultivation is at 26 ° C (± 1 ° C) and pH 6.5.

**3.** The process as claimed in claim 1 or 2, wherein the cultivation is carried out for 90 hours.

**4.** The process as claimed in one of claims 1 to 3, wherein the fermentation is carried out as a submerged fermentation.

**5.** A process for preparing pharmaceuticals, which comprises a compound of the formula I as claimed in claim 1, together with customary auxiliaries and/or vehicles, being converted into a suitable administration form.

**6.** The use of a compound as claimed in claim 1 for the preparation of pharmaceuticals having an antibiotic action.

**Revendications**
**Revendications pour les Etats contractants suivants : AT, BE, CH, DE, FR, GB, IT, LI, LU, NL, SE**

**1.** Composé de formule:

```
H3C(CH2)5-CH(CH2)6-CH=CH-CH-CH-CH-CH-COOH   .
          |                 |  |   |  |
          OH                OH OAcOH NH2
```

**2.** Procédé pour préparer le composé selon la revendication 1, caractérisé en ce que l'on effectue la culture du champignon Aspergillus fumigatus No. Y-83 045 (DSM 4152) dans des conditions aérobies, sur un milieu nutritif contenant des sources de carbone, des sources d'azote, des sels inorganiques et des oligo-éléments, à une température comprise entre 24 et 30 ° C et à un pH compris entre 6,0 et 8,0, pendant 66 à 96 heures, et on isole le composé du bouillon de culture et on le purifie de façon habituelle.

**3.** Procédé selon la revendication 2, caractérisé en ce que l'on effectue la culture à 26 ° C (± 1 ° C) et à pH 6,5.

**4.** Procédé selon la revendication 2 ou 3, caractérisé en ce que l'on effectue la culture pendant 90 heures.

**5.** Procédé selon l'une des revendications 2 à 4, caractérisé en ce que l'on effectue la fermentation sous forme de fermentation immergée.

6. Médicament caractérisé en ce qu'il contient un composé selon la revendication 1, ainsi que des adjuvants et/ou des véhicules usuels.

7. Utilisation d'un composé selon la revendication 1 pour préparer des médicaments ayant une action antibiotique.

8. Aspergillus fumigatus Y-83 0405 (DSM 4152).

**Revendications pour les Etats contractants suivants : ES, GR**

1. Procédé pour préparer un composé de formule I:

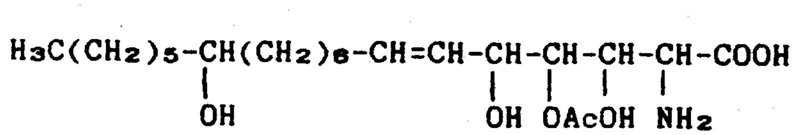

caractérisé en ce que l'on effectue la culture du champignon Aspergillus fumigatus No. Y-83 045 (DSM 4152) dans des conditions aérobies, sur un milieu nutritif contenant des sources de carbone, des sources d'azote, des sels inorganiques et des oligo-éléments, à une température comprise entre 24 et 30 ° C et à un pH compris entre 6,0 et 8,0, pendant 66 à 96 heures, et on isole le composé du bouillon de culture et on le purifie de façon habituelle.

2. Procédé selon la revendication 1, caractérisé en ce que l'on effectue la culture à 26 ° C (± 1 ° C) et à pH 6,5.

3. Procédé selon la revendication 1 ou 2, caractérisé en ce que l'on effectue la culture pendant 90 heures.

4. Procédé selon l'une des revendications 1 à 3, caractérisé en ce que l'on effectue la fermentation sous forme de fermentation immergée.

5. Procédé pour préparer un médicament, caractérisé en ce que l'on met un composé de formule I selon la revendication 1 sous une forme de présentation appropriée, avec des adjuvants et/ou des véhicules usuels.

6. Utilisation d'un composé selon la revendication 1 pour préparer des médicaments ayant une action antibiotique.